# EUROPEAN PATENT APPLICATION

(11) **EP 3 618 456 A1**
(43) Date of publication of application: **04.03.2020**
(21) Application number: 18191457.3
(22) Date of filing: 29.08.2018
(51) Int. Cl.: H04R 25/00, A61B 5/16

(54) **OWN VOICE SIGNAL PROCESSING METHOD**

(71) Applicant: Oticon A/S, 2765 Smørum (DK)
(72) Inventor: PONTOPPIDAN, Niels Henrik, DK-2765 Smørum (DK); LAUGESEN, Søren, DK-5500 Middelfart (DK); NAYLOR, Graham, DK-3070 Snekkersten (DK); MÖHRING, Finn, DK-2765 Smørum (DK); JENSEN, Niels Søgaard, DK-3450 Allerød (DK); LUNNER, Thomas, DK-2765 Smørum (DK); PETERSEN, Eline Borch, DK-3000 Helsingør (DK); LAPLANTE-LÉVESQUE, Ariane, DK-2765 Smørum (DK); CHRISTIANSEN, Thomas Ulrich, DK-2765 Smørum (DK)
(74) Representative: William Demant

(57) **Abstract**

A signal processing method and a hearing device for isolating a user's own voice from microphone signals is disclosed. The signal processing method comprises the following steps. Providing at least two microphones at one ear of a user or at least one microphone at each ear of the user. Receiving sound signals by the microphones. Isolating a user's own voice from the microphone signals by way of a combination of at least two of the following processes: a) applying own voice detectors to the microphone signals, b) using monaural and binaural directional microphone systems, c) using monaural and binaural directional microphone systems with near field emphasis, and d) applying speaker identification models to the microphone signals. Estimating a stress level or cognitive load from the isolated voice. Adjusting settings of a device in dependence of the estimated stress level or cognitive load.

## Description

### FIELD

The present disclosure relates to a signal processing method and a hearing device. More particularly, the disclosure relates to a signal processing method and a hearing device for isolating a user's own voice from microphone signals.

### BACKGROUND

Interaction between a user and hearing devices such as hearing aids in order to control the functions of the hearing device is currently limited to control interfaces such as the buttons on the actual hearing device, buttons and sliders on auxiliary devices such as hearing device remote controls and more sophisticated screen menus on auxiliary devices such as smartphones and tablet PCs.

The average life expectancy has been increasing for many years. Unfortunately, health, mental health and age is often accompanied with different cognitive changes.

A stroke is characterized by poor blood flow or bleeding in the brain resulting in brain cell death. Strokes are extremely common and "Lifestyle disease" such as diabetes and obesity are risk factors for strokes: as the prevalence of lifestyle diseases continues to increase, so will the incidence of strokes. Strokes have important consequences ranging from permanent physical and cognitive impairment to death. Early detection of a stroke is paramount to reduce its negative consequences. Treatment such as anti-clot medication or surgery has much better chances of success if started early, before significant brain cell death occurs. The current problem is that most strokes are detected and treated too late.

Changes in voice/speech/language is an early warning sign of a stroke. A health professional in Canada once suffered a stroke whilst presenting to her colleagues in the hospital auditorium where she worked. Her colleagues quickly detected that she was suffering a stroke as her articulation of words became imprecise and as she started looking for words and using semantically and grammatically incorrect words in her presentation.

Mental-health disorders, like depression and anxiety (or conditions, like stress, which may lead to such mental-health disorders) may develop very slowly over a long period of time.

Due to the slow development and the fact that symptoms (at an early stage) may be quite subtle, the disorders may not be discovered until a stage where treatment is much more difficult than it would have been if the disorder had been detected and diagnosed at an earlier stage.

One symptom of many mental-health disorders is a change in social behaviour. This change may, among other things, be reflected in the speech produced by the person affected by the disorder, both in terms of its linguistic (semantic) and acoustic contents. For example, a person who is developing a depression may be less talkative, use more negatively loaded words, and speak with an angrier intonation (or with less intonation), compared to the 'normal' condition. These changes may (or may not) be (subjectively) noticed by family, friends, colleagues, or the person him- or herself.

Thus, a continuous recording (e.g. made through the microphone in a hearing device) and a subsequent acoustic and linguistic analysis of the speech spoken by a person may show a long-term trend away from the 'normal' pattern, indicating that a mental-health disorder may be under development. The prediction may be even more accurate if combined with output from other sensors attached to the body, e.g. one or more EEG electrodes (measuring brain activity, which could be integrated in the hearing device.

Dementia is one age related decease and has no cure, but if diagnosed early, the progress can be delayed and with careful monitoring, medication can be continuously optimized. At present day, the diagnostics require doctors to monitor potential patients often at in-clinic visits with irregular follow-up visits. Dementia-patients are known to have increased percentage of pauses while performing simple tasks, lower voice level quality (pitch, jitter, and shimmer), less speech continuity and fluency and finally lower vocabulary richness. A large proportion of elderly people also suffers from hearing impairment and often wears a hearing aid to solve this problem.

Using EEG-sensors at the hearing device worn by the user further allows the user to interact with the hearing device by estimating a stress level or cognitive load of the user. Wearing EEG-sensors is, however, inconvenient for the user.

Therefore, there is a need to provide a solution that allows for conveniently estimating a stress level or cognitive load of the user at the hearing device.

### SUMMARY

According to an aspect, a signal processing method comprises the following steps. Providing at least two microphones at one ear of a user or at least one microphone at each ear of the user. Receiving sound signals by the microphones. Isolating a user's own voice from the microphone signals by way of a combination of at least two of the following processes: a) applying own voice detectors to the microphone signals, b) using monaural and binaural directional microphone systems, c) using monaural and binaural directional microphone systems with near field emphasis, and d) applying speaker identification models to the microphone signals. Estimating a stress level or cognitive load from the isolated voice. Adjusting settings of a device in dependence of the estimated stress level or cognitive load.

The signal processing method according to the disclosure allows for using microphones instead of EEG-sensors. This is more convenient for the user, as the user does not need to wear EEG-sensors. Instead microphones, e.g., already present in a hearing device worn by the user, can be used in order to receive sound signals. Using microphones thus also allows for using a hearing device for estimating stress level or cognitive load of the user. Estimating the stress level or cognitive load of the user is particularly important in difficult listening situations. For hearing-impaired listeners these are listening situations when there is more than one talker or reverberation. Isolating a user's own voice from the microphone signals before estimating the stress level or cognitive load allows for essentially disregarding voices of other speakers when the stress level or cognitive load of the user is estimated. This increases the reliability of the estimated stress level or cognitive load, particularly in difficult listening situations.

The stress level or cognitive load can for example be estimated from pitch, frequency, amplitude, speaking rate, variability of pitch, variability of frequency, variability of amplitude, and/or variability of speaking rate of the isolated voice of the user.

The step of isolating a user's own voice from the microphone signals can also combine three or all of the processes a), b), c), and d). The signal processing method can also provide two or more microphones, e.g. three microphones at each ear of the user. Two microphones at one ear can for example be arranged in a front-rear configuration, i.e., one front microphone and one rear microphone or both microphones can be arranged as front or rear microphones. The front microphone is arranged at the front of the user's ear and the rear microphone is arranged at the rear of the user's ear. A microphone can also be arranged between the tympanic membrane of the ear of the user and an output transducer, e.g. loudspeaker, of the hearing device. The arrangement between the tympanic membrane and a loudspeaker results in a smaller attenuation of the frequency range of speech signals than the high frequency range. Thus this arrangement leads to better sound signals for the estimation of a user's frequency modulation (FM) and amplitude modulation (AM) during speech. For three microphones for example two front microphones can be used in combination with one rear microphone, two rear microphones can be used with one front microphone or the microphones can be arranged in a linear array. The microphone signals of the microphones can be transmitted between the ears or to an auxiliary device arranged at the user in order to process the microphone signals, e.g., by using a binaural link between two hearing devices or a wired or wireless links between the hearing devices and the auxiliary device.

According to an embodiment of the signal processing method the process a) of applying own voice detectors to the microphone signals comprises one or both of the following processes A) and B). The process A) comprises the following steps. Determining from the microphone signals the characteristics resulting from the microphones being in the acoustical near-field of the user's mouth and in the far-field of other sources of sound. Assessing based on these characteristics whether the sound signals originate from the user's own voice or originate from another source. The process B) comprises the following steps. Determining from the microphone signals the characteristics resulting from the user's mouth being placed symmetrically with respect to the microphones arranged at the user's head. Assessing based on these characteristics whether the sound signals originate from the user's own voice or originate from another source. The process a) of applying own voice detectors to the microphone signals further comprises providing a binary flag that represents whether the user's own voice is detected in the microphone signals or a probability value that represents the probability of the user's own voice being present in the microphone signals.

The embodiment of the signal processing method allows for improved reliability in own voice detection.

The characteristics resulting from the microphones being in the acoustical near-field of the user's mouth and in the far-field of other sources of sound determined in process A) can for example be determined by the following steps. Filtering the microphone signals with FIR filters, the filter coefficients of which are determined so as to maximize the difference in sensitivity towards sound coming from the mouth of the user as opposed to sound coming from all directions by using a Mouth-to-random-far-field index (abbreviated M2R). Comparing the M2R obtained by using only one microphone at each ear of the user compared with the M2R obtained by using more than one microphone at each ear of the user in order to take into account the different source strengths pertaining to the different acoustic sources.

This allows for taking advantage of the acoustic near field close to the mouth of the user.

The characteristics resulting from the user's mouth being placed symmetrically with respect to the microphones arranged at the user's head determined in process B) can for example be determined by the following steps. Receiving microphone signals x₁(n) and x₂(n), from microphones positioned at each ear of the user. Computing the cross-correlation function between the two microphone signals: Rₓ₁ₓ₂(k)=E{x₁(n)x₂(n-k)}. Applying a detection criterion to the output Rₓ₁ₓ₂(k), such that if the maximum value of Rₓ₁ₓ₂(k) is found at k=0 the dominating sound source is in the median plane of the user's head and may thus likely be own voice, whereas if the maximum value of Rₓ₁ₓ₂(k) is found elsewhere the dominating sound source is away from the median plane of the user's head and cannot be own voice.

This allows for utilizing the similarities of the sound signals received by the microphones on the two sides of the head when the sound source is the user's own voice.

Process a) can also be applied in an embodiment of the signal processing method with a decimated polyphase filterbank, wherein the binary flag that represents whether the user's own voice is detected in the microphone signals or the probability value that represents the probability of the user's own voice being present in the microphone signals may apply for all time-frequency bins at the designated time intervals. The own voice detection can also be applied in the filterbank channels, such that it provides an output for each time-frequency bin.

In one embodiment of the signal processing method a binaural link is provided between hearing devices at each ear of the user for exchanging information such as own voice detection information, e.g., binary flag or probability of detection of user's own voice. The binaural link can be wired or wireless, e.g. via Bluetooth. The hearing devices can use the information in order to determine an overall probability of detection of user's own voice or whether the user's own voice is present in both microphone signals or not. Furthermore, computations can be divided between the hearing devices such that one hearing device isolates the user's own voice from the microphone signals while the other hearing device estimates the stress level or cognitive load from the isolated voice.

Using a binaural link allows for improving the performance of the own voice detection by fusion of user's own voice detection signals, e.g. binary flag or probability of detection of user's own voice and user's own voice detection outcomes at each ear of the user. Furthermore, the use of a binaural link allows for improving the processing of the microphone signals, as work load can be divided between two hearing devices.

According to another embodiment of the signal processing method the process of using monaural and binaural directional microphone systems comprises attenuating sound signals that do not have a time delay between the at least two microphones at one ear of the user that matches the time delay from the user's mouth direction, whereby a directional beam is generated.

This allows for deriving directional beams in the user's mouth direction at each ear of the user.

According to yet another embodiment of the signal processing method the process of using monaural and binaural directional microphone systems further comprises attenuating out of phase signals in two directional beams, each of which is generated from attenuating the sound signals at one ear of the user, in a binaural beam, such that the binaural beam as a result points directly forward from a mouth position of the user.

The binaural beam points directly forward from a mouth position of the user, i.e., where user's own voice is likely produced and thus allows for improved detection of the user's own voice.

In order to generate the binaural beam, the two directional beams can be transmitted via the binaural link from one ear of the user to the other. The two directional beams are used to construct the binaural beam which points directly forward. Only signals that originate from the junction of the three beams pass through the combined binaural and monaural directional microphone system located at the mouth position. Therefore, signals derived using the combined binaural and monaural directional microphone system are likely to comprise user's own voice. Other signal separation methods known to the person skilled in the art can be applied to improve the monaural and binaural microphone systems.

According to a further embodiment of the signal processing method the process of using monaural and binaural directional microphone systems with near field emphasis comprises the following steps. Extracting acoustical parameters from the microphone signals that allow to decide on whether the sound signals originate from a position close to the microphones or far from the microphones. Using the information whether the sound signals originate from a position close to the microphones or far from the microphones to isolate the user's own voice.

This allows for isolating the user's own voice without using the binaural link.

According to yet another embodiment of the signal processing method the process of applying speaker identification models to the microphone signals comprises the following steps. Providing an output for each time-frequency bin of the microphone signals. Selecting only the time-frequency bins that resemble speech characteristics of the user for further processing of the microphone signals.

This allows for improving the isolation of the user's own voice as only time-frequency bins are considered for the signal processing that are relevant for user's own voice.

According to another embodiment the signal processing method comprises the following steps. Deriving speech characteristics of the user from the isolated voice. Storing the speech characteristics of the user for subsequent use.

This allows for learning the speech characteristics, e.g., tempo-spectral change patterns, of the user over time. The stored speech characteristics can then be used for an improved identification of the voice of the user in a set of voices, e.g. for voice activation or isolating the user's own voice.

The speech characteristics of the user can for example comprise pitch, frequency, amplitude, speaking rate, variability of pitch, variability of frequency, variability of amplitude, and/or variability of speaking rate of the isolated voice of the user in relaxed and stressed condition. The stored speech characteristics can be used in order to improve the estimate of the stress level or cognitive load of the user. In one embodiment of the signal processing method the stored speech characteristics of the user are used in the step of estimating the stress level or cognitive load from the isolated voice.

According to yet another embodiment of the signal processing method a combination of at least two of the processes performed on the microphone signals is done by the following steps. Fusing the outputs of the processes. Extracting frequency modulations and amplitude modulations. Furthermore, the step of estimating of the stress level or cognitive load from the isolated voice is done by estimating the stress level or cognitive load from the extracted frequency modulations and amplitude modulations.

This allows for an improved estimation of the stress level or cognitive load of the user.

Each of the processes performed on the microphone signals provides either an isolated voice signal, an estimate for the probability of the user's own voice being present in the microphone signals, a binary flag whether the user's own voice is present in the microphone signals or for example time frequency bins in a decimated polyphase filterbank setup where the user's signal dominates. Processing the signals together allows for improved extraction of the frequency modulation and amplitude modulation of the user's speech and thus for improved estimating the cognitive load or stress level. Fusing more signals also lowers possible influences of noise, e.g., speech of other talkers or sounds from other acoustic or sound sources.

According to a further embodiment the signal processing method comprises the following steps. Providing at least a microphone at a position between a loudspeaker and tympanic membrane of the user. Using the sound signals received by the at least one microphone at a position between loudspeaker and tympanic membrane of the user for isolating a user's own voice.

This allows for an improved estimation of the frequency modulation and amplitude modulation during speech of the user, as the frequency range of speech signals in the microphone signals is less attenuated than the high frequency range for a microphone at the position between loudspeaker and tympanic membrane.

According to one embodiment the signal processing method comprises the following steps. Monitoring a heart rate of the user which comprises receiving sound signals by the microphones and estimating the heart rate of the user based on the microphone signals. Estimating a heart-rate-variability of the user from the heart rate of the user. Estimating the stress level or cognitive load based on the heart-rate-variability of the user.

According to another embodiment the signal processing method comprises the following steps. Monitoring a heart rate of the user which comprises receiving sound signals by the microphones and estimating the heart rate of the user based on the microphone signals. The microphones used to estimate the heart rate of the user are arranged at a position between a loudspeaker and tympanic membrane of the user. The signal processing method further comprises the following steps. Estimating a heart-rate-variability of the user from the heart rate of the user. Estimating the stress level or cognitive load based on the heart-rate-variability of the user.

This allows for improving the estimation of the stress level or cognitive load, as the heart-rate originates from the user and no isolation of the user's own voice is necessary.

The embodiments of estimating the stress level or cognitive load from the isolated voice can be combined with the embodiments of estimating the stress level or cognitive load from the heart-rate-variability of the user in order to further improve the estimate of the stress level or cognitive load, as more parameters are regarded.

According to another aspect, a hearing device comprises at least two microphones adapted to be arranged at one ear of a user or at least one microphone adapted to be arranged at each ear of the user, and a signal processing unit. The microphones are for receiving sound signals. The signal processing unit is adapted to isolate the user's own voice from the microphone signals by way of a combination of at least two of the following processes: a) applying own voice detectors to the microphone signals, b) using monaural and binaural directional microphone systems, c) using monaural and binaural directional microphone systems with near field emphasis, and d) applying speaker identification models to the microphone signals. The signal processing unit is further adapted to estimate a stress level or cognitive load from the isolated voice. Furthermore, the signal processing unit is adapted to adjust settings of the hearing device in dependence of the estimated stress level or cognitive load.

The hearing device according to the disclosure allows for improved estimation of the stress level or cognitive load. As the settings of the hearing device are adjusted in dependence of the estimated stress level or cognitive load the hearing device allows for an improved listening experience, as the hearing device can automatically adapt to the stress level or cognitive load of the user.

According to an embodiment of the hearing device the signal processing unit is adapted to apply own voice detectors to the microphone signals by performing one or both of the following processes A) and B). The process A) comprises the following steps. Determining from the microphone signals the characteristics resulting from the microphones being in the acoustical near-field of the user's mouth and in the far-field of other sources of sound. Assessing based on these characteristics whether the sound signals originate from the user's own voice or originate from another source. The process B) comprises the following steps. Determining from the microphone signals the characteristics resulting from the user's mouth being placed symmetrically with respect to the microphones arranged at the user's head. Assessing based on these characteristics whether the sound signals originate from the user's own voice or originate from another source. The signal processing unit is further adapted to provide a binary flag that represents whether the user's own voice is detected in the microphone signals or a probability value that represents the probability of the user's own voice being present in the microphone signals.

According to another embodiment the hearing device comprises a memory adapted for storing a user's speech characteristics resembling a selection of certain time-frequency bins characteristic for the user's speech and storing a set of initial speech characteristics.

This allows for improved isolation of the user's own voice over time, as the speech characteristics for the user are learned over time. The initial speech characteristics can be selected for example from a set of initial speech characteristics, e.g., young man, old man, young woman, old woman, high voice, low voice, or a set of parameters.

According to yet another embodiment the hearing device comprises a transmitter for transmitting signals to another hearing device or an auxiliary device and a receiver for receiving signals from another hearing device or an auxiliary device.

This allows for dividing the computation across several devices and thus can improve the speed of the computation.

According to yet another aspect, a hearing system comprises at least one hearing device and at least one auxiliary device or at least two hearing devices and is adapted to perform the signal processing method. Each auxiliary device comprises a transmitter, a receiver and a signal processing unit. The transmitter is for transmitting signals to the hearing device. The receiver is for receiving signals from the hearing device. The signal processing unit is for processing signals received from the hearing device.

The auxiliary devices can have increased memory and CPU power, as well as a larger energy source than the hearing device. The hearing devices can transmit microphone signals to the auxiliary device in order to perform the calculations on the auxiliary device. By performing calculations on the auxiliary device, the power consumption of the hearing devices can be reduced. For example, the isolation of the user's own voice can be performed on the auxiliary device. The auxiliary device can also have microphones in order to receive sound signals from the surroundings. In this case the auxiliary device does not need to rely on the transmission of the microphone signals from the hearing devices, but can also receive additional microphone signals from them in order to improve the isolation of the user's own voice. Parts of the signal processing method according to the disclosure can be performed on the auxiliary device and settings of the auxiliary device as well as the hearing devices can then be adjusted in dependence of the stress level or cognitive load as estimated by the auxiliary device.

The hearing system allows for utilizing the microphones of the hearing devices or of an auxiliary device in order to determine the stress level or cognitive load of the user. The settings of the hearing devices and auxiliary device can be adjusted in dependence of the estimated stress level. For example, if the user is in a stressed state the sound level at the hearing devices can be decreased and an audio file intended to help the user to relax can be selected at the auxiliary device or an audio file currently playing on the auxiliary device can be stopped. The determined stress level or cognitive load of the user can be transferred to the user's general practitioner or family member in order to continuously monitoring the cognitive health of the user.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
Figure 1 illustrates an embodiment of a hearing system with two hearing devices according to the disclosure;
Figure 2 illustrates directional beam forming according to the disclosure;
Figure 3 illustrates binaural beam forming according to the disclosure;
Figure 4 illustrates an embodiment of a hearing system with an auxiliary device and two hearing devices according to the disclosure;
Figure 5 illustrates an embodiment of a hearing system with an auxiliary device and two hearing devices according to the disclosure;
Figure 6 illustrates a hearing device 10, 10' of a hearing system 100' according to an embodiment of the disclosure;
Figure 7 illustrates an example according to an embodiment of the disclosure
Figure 8 illustrates a stroke detection system according to an embodiment of the disclosure;
Figure 9 illustrates an embodiment of the signal processing method according to the disclosure;
Figure 10 illustrates an embodiment of the signal processing method according to the disclosure;
Figure 11 illustrates an embodiment of the signal processing method according to the disclosure; and
Figure 12 illustrates an embodiment of the signal processing method according to the disclosure.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include microprocessors, microcontrollers, digital signal processors (DSPs), field programmable gate arrays (FPGAs), programmable logic devices (PLDs), gated logic, discrete hardware circuits, and other suitable hardware configured to perform the various functionality described throughout this disclosure. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

A hearing device may include a hearing aid that is adapted to improve or augment the hearing capability of a user by receiving an acoustic signal, e.g. sound signal, from a user's surroundings, generating a corresponding audio signal, e.g. microphone signal, possibly modifying the audio signal and providing the possibly modified audio signal as an audible signal to at least one of the user's ears. The "hearing device" may further refer to a device such as an earphone or a headset adapted to receive an audio signal electronically, possibly modifying the audio signal and providing the possibly modified audio signals as an audible signal to at least one of the user's ears. Such audible signals may be provided in the form of an acoustic signal radiated into the user's outer ear, or an acoustic signal transferred as mechanical vibrations to the user's inner ears through bone structure of the user's head and/or through parts of middle ear of the user or electric signals transferred directly or indirectly to cochlear nerve and/or to auditory cortex of the user.

The hearing device is adapted to be worn in any known way. This may include i) arranging a unit of the hearing device behind the ear with a tube leading air-borne acoustic signals into the ear canal or with a receiver/ loudspeaker arranged close to or in the ear canal such as in a Behind-the-Ear type hearing aid, and/ or ii) arranging the hearing device entirely or partly in the pinna and/ or in the ear canal of the user such as in an In-the-Ear type hearing aid or In-the-Canal/ Completely-in-Canal type hearing aid, or iii) arranging a unit of the hearing device attached to a fixture implanted into the skull bone such as in Bone Anchored Hearing Aid or Cochlear Implant, or iv) arranging a unit of the hearing device as an entirely or partly implanted unit such as in Bone Anchored Hearing Aid or Cochlear Implant.

A "hearing system" refers to a system comprising one or two hearing devices, and a "binaural hearing system" refers to a system comprising two hearing devices where the devices are adapted to cooperatively provide audible signals to both of the user's ears. The hearing system or binaural hearing system may further include auxiliary device(s) that communicates with at least one hearing device, the auxiliary device affecting the operation of the hearing devices and/or benefitting from the functioning of the hearing devices. A wired or wireless communication link between the at least one hearing device and the auxiliary device is established that allows for exchanging information (e.g. control and status signals, possibly audio signals) between the at least one hearing device and the auxiliary device. Such auxiliary devices may include at least one of remote controls, remote microphones, audio gateway devices, mobile phones, public-address systems, car audio systems or music players or a combination thereof. The audio gateway is adapted to receive a multitude of audio signals such as from an entertainment device like a TV or a music player, a telephone apparatus like a mobile telephone or a computer, a PC. The audio gateway is further adapted to select and/or combine an appropriate one of the received audio signals (or combination of signals) for transmission to the at least one hearing device. The remote control is adapted to control functionality and operation of the at least one hearing devices. The function of the remote control may be implemented in a Smartphone or other electronic device, the Smartphone/ electronic device possibly running an application that controls functionality of the at least one hearing device.

In general, a hearing device includes i) an input unit such as a microphone for receiving an acoustic signal from a user's surroundings and providing a corresponding input audio signal, e.g. microphone signal, and/or ii) a receiving unit for electronically receiving an input audio signal. The hearing device further includes a signal processing unit for processing the input audio signal and an output unit for providing an audible signal to the user in dependence on the processed audio signal.

The input unit may include multiple input microphones, e.g. for providing direction-dependent audio signal processing. Such directional microphone system is adapted to enhance a target acoustic source among a multitude of acoustic sources in the user's environment. In one aspect, the directional system is adapted to detect (such as adaptively detect) from which direction a particular part of the microphone signal originates. This may be achieved by using conventionally known methods. The signal processing unit may include amplifier that is adapted to apply a frequency dependent gain to the input audio signal. The signal processing unit may further be adapted to provide other relevant functionality such as compression, noise reduction, etc. The output unit may include an output transducer such as a loudspeaker/ receiver for providing an air-borne acoustic signal transcutaneously or percutaneously to the skull bone or a vibrator for providing a structure-borne or liquid-borne acoustic signal. In some hearing devices, the output unit may include one or more output electrodes for providing the electric signals such as in a Cochlear Implant.

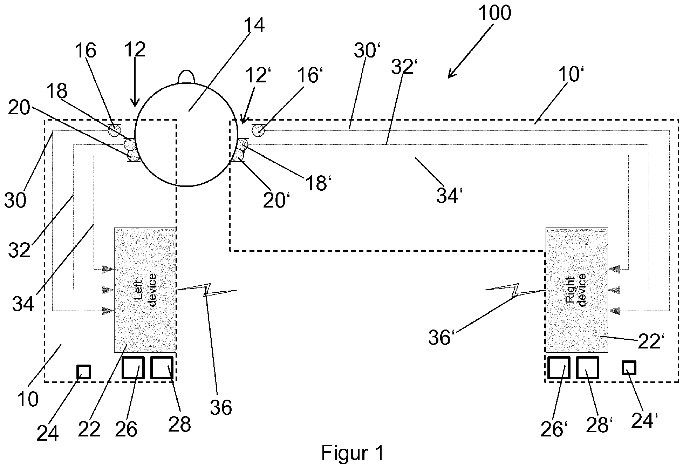

Now referring to figure 1, which illustrates an embodiment of a hearing system 100 with two hearing devices 10, 10' according to an aspect of the disclosure. The left hearing device 10 is arranged at the left ear 12 of the user 14 and the right hearing device 10' is arranged at the right ear 12' of the user 14. The depiction of larger sizes of the hearing devices 10, 10' compared to the user 14 are for illustrative purposes.

Each hearing device 10, 10' comprises three microphones 16, 18, 20, 16', 18', 20', a signal processing unit 22, 22', a memory 24, 24', a receiver 26, 26', and a transmitter 28, 28' and output transducers (not shown). Output transducers can for example be loudspeakers, vibrators or one or more output electrodes.

Each of the microphones 16, 18, 20, 16', 18', 20' receives sound signals and generates microphone signals 30, 32, 34, 30', 32', 34' from the sound signals. The microphone signals 30, 32, 34 of the left hearing device 10 are provided to the signal processing unit 22. The microphone signals 30', 32', 34' of the right hearing device 10' are provided to the signal processing unit 22'. The signal processing units 22, 22' can comprise filter banks in order to separate the microphone signals into sub-band signals divided over time-frequency bins (not shown).

The microphone signals 30', 32', 34' of the right hearing device 10' can also be transmitted to the left hearing device 10 using binaural link 36' between transmitter 28' and receiver 26. The microphone signals 30, 32, 34 of the left hearing device 10 can also be transmitted to the right hearing device 10' using binaural link 36 between transmitter 28 and receiver 26'. This allows a binaural operation of the hearing system 100.

The signal processing units 22, 22' isolate the user's own voice from the microphone signals 30, 32, 34, 30', 32', 34'. Therefore, the signal processing units 22, 22' perform at least two of the following processes a) applying own voice detectors to the microphone signals, b) using monaural and binaural directional microphone systems, c) using monaural and binaural directional microphone systems with near field emphasis, and d) applying speaker identification models to the microphone signals.

Optionally the signal processing units 22, 22' can derive speech characteristics, e.g., tempo-spectral change patterns, of the user from the isolated voice and store them in the memories 24, 24'. The speech characteristics of the user can for example comprise pitch, frequency, amplitude, speaking rate, variability of pitch, variability of frequency, variability of amplitude, and/or variability of speaking rate of the isolated voice of the user in relaxed and stressed condition. The stored speech characteristics can be used in order to improve the estimate of the stress level or cognitive load of the user, e.g., by selecting only certain time-frequency bins of the microphone signals 30, 32, 34, 30', 32', 34' which are characteristic for the user's speech.

The outputs of the processes a), b), c), and d), which were performed by the signal processing units 22, 22', are then combined by the signal processing units 22, 22' in order to generate an isolated voice signal. Each of the signal processing units 22, 22' can for example also perform two of the processes a), b), c), and d) and the outputs can be transmitted to the other hearing device 10 and 10', respectively, via the binaural link 36, 36' in order to combine the outputs of, e.g., all four processes a), b), c), and d). Using a combination of processes for isolating the user's own voice improves the reliability of the obtained isolated voice signal.

The signal processing units 22, 22' then estimate a stress level or cognitive load from the isolated voice. The estimation of the stress level or cognitive load from the isolated voice can further be combined with other estimations of the stress level or cognitive load from other sources, e.g., from heart-rate-variability, sweat production, extracted frequency modulations of the user's own voice, extracted amplitude modulations of the user's own voice or the like (not shown). This allows for an even more improved and more reliable estimation of the stress level or cognitive load of the user 14.

The signal processing units 22, 22' can also fuse the outputs of the processes a), b), c), and d) by extracting frequency modulations and amplitude modulations. The stress level or cognitive load is estimated from the extracted frequency modulations and amplitude modulations in this case.

The estimated stress level or cognitive load is then used by the signal processing units 22, 22' to adjust settings of the hearing devices 10, 10'. Each of the performed operations in order to estimate the stress level or cognitive load can also be performed only by one of the signal processing units 22, 22' and the output can then be transmitted via the binaural link 36, 36' to the other hearing device 10, 10'.

The signal processing units 22, 22' can apply own voice detectors to the microphone signals 30, 32, 34, 30', 32', 34', i.e., process a), by performing one or both of the following processes A) and B). Process A) comprises determining from the microphone signals the characteristics resulting from the microphones being in the acoustical near-field of the user's mouth and in the far-field of other sources of sound, and assessing based on these characteristics whether the sound signals originate from the user's own voice or originate from another source. Process B) comprises determining from the microphone signals the characteristics resulting from the user's mouth being placed symmetrically with respect to the microphones arranged at the user's head, and assessing based on these characteristics whether the sound signals originate from the user's own voice or originate from another source.

The characteristics resulting from the microphones 16, 18, 20, 16', 18', 20' being in the acoustical near-field of the user's mouth 40 and in the far-field of other sources of sound determined in process A) are determined by the following steps. Filtering the microphone signals 30, 32, 34, 30', 32', 34' with FIR filters in the signal processing units 22, 22', the filter coefficients of which are determined so as to maximize the difference in sensitivity towards sound coming from the mouth 40 of the user 14 as opposed to sound coming from all directions by using a Mouth-to-random-far-field index (abbreviated M2R). Comparing the M2R obtained by using only the front microphone 16, 16' at each ear 12, 12' of the user 14 compared with the M2R obtained by using all three microphones 16, 18, 20, 16', 18', 20' at each ear 12, 12' of the user 14 in order to take into account the different source strengths pertaining to the different acoustic sources.

The characteristics resulting from the user's mouth being placed symmetrically with respect to the microphones 16, 16' arranged at the user's head determined in process B) are determined by the following steps. Receiving microphone signals x₁(n) 30 and x₂(n) 30', from microphones 16, and 16' positioned at each ear 12, 12' of the user 14 by the signal processing units 22, 22'. Computing the cross-correlation function between the two microphone signals 30, 30': Rₓ₁ₓ₂(k)=E{x₁(n)x₂(n-k)} in the signal processing units 22, 22'. Applying a detection criterion to the output Rₓ₁ₓ₂(k) in the signal processing units 22, 22', such that if the maximum value of Rₓ₁ₓ₂(k) is found at k=0 the dominating sound source is detected as being in the median plane of the user's head and may thus likely be own voice, whereas if the maximum value of Rₓ₁ₓ₂(k) is found elsewhere the dominating sound source is detected to be away from the median plane of the user's head and cannot be own voice.

Based on the results of process A) or B) the signal processing units 22, 22' can provide a binary flag that represents whether the user's own voice is detected in the microphone signals 30, 32, 34, 30', 32', 34' or a probability value that represents the probability of the user's own voice being present in the microphone signals 30, 32, 34, 30', 32', 34'. The binary flag or probability obtained by process a) can be used in order to improve the isolation of the user's own voice. For example, time-frequency bins that do not contain user's own voice or have a probability below a threshold probability value can be disregarded for further processing. Therefore, the signal processing units 22, 22' can perform process a) using a decimated polyphaser filter bank, wherein the binary flag that represents whether the user's own voice is detected in the microphone signals or the probability value that represents the probability of the user's own voice being present in the microphone signals may apply for all time-frequency bins at the designated time intervals. The signal processing units 22, 22' can also apply the own voice detection in the filter bank channels, such that it provides an output for each time-frequency bin.

The signal processing units 22, 22' can perform the process of applying speaker identification models to the microphone signals 30, 32, 34, 30', 32', 34', i.e., process b), by performing the following steps. Providing an output for each time-frequency bin of the microphone signals 30, 32, 34, 30', 32', 34' using the filterbank in the signal processing units 22, 22'. Selecting only the time-frequency bins that resemble speech characteristics of the user for further processing of the microphone signals.
The speech characteristics, e.g. tempo-spectral change patterns, of the user are stored in the memories 24, 24'. There can be an initial set of speech characteristics and the memory can store the characteristics of the user when the user speaks. Therefore, the signal processing units 22, 22' can derive speech characteristics of the user 14 from the isolated voice and store the speech characteristics of the user 14 for subsequent use.

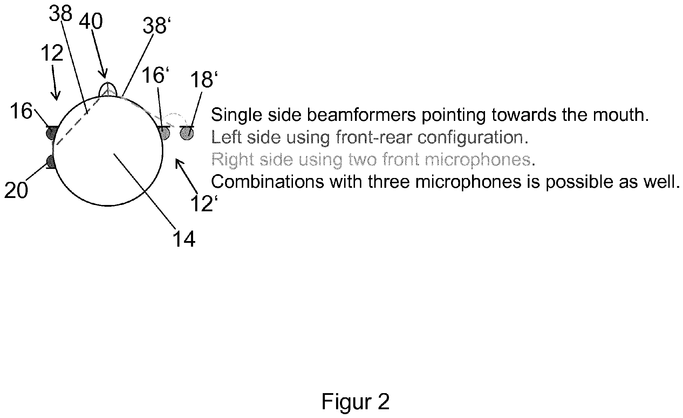

Figure 2 illustrates directional beam forming according to an embodiment of the disclosure. The front microphone 16 and rear microphone 20 at the left ear 12 of user 14 are arranged in a front-rear configuration. The two front microphone 16' and intermediate microphone 18' at the right ear 12' of user 14 are arranged in a two front microphone configuration. The microphones 16, 18, 20, 16', 18', 20' can be arranged at, on, behind, or in the ear 12, 12'. Furthermore, the microphones 16, 18, 20, 16', 18', 20' can also be arranged between the tympanic membrane and the output transducer of the hearing device 10, 10', e.g. in order to measure a heart rate of the user 14.

A directional beam 38 of the microphones 16, 20 at the left ear 12 is generated by attenuating sound signals that do not have a time delay between the microphones 16 and 20 that matches the time delay from the user's mouth direction. A directional beam 38' of the microphones 16', 18' at the right ear 12' is generated by attenuating sound signals that do not have a time delay between the microphones 16' and 18' that matches the time delay from the user's mouth direction. Thus the directional beams 38 and 38' point towards the mouth 40 of the user 14. Each of the two different microphone configurations generates a directional beam 38, 38'.

It is also possible to perform directional beam forming using three microphones, e.g., microphones 16, 18, 20, 16', 18', 20' of the hearing devices 10, 10' shown in Fig. 1.

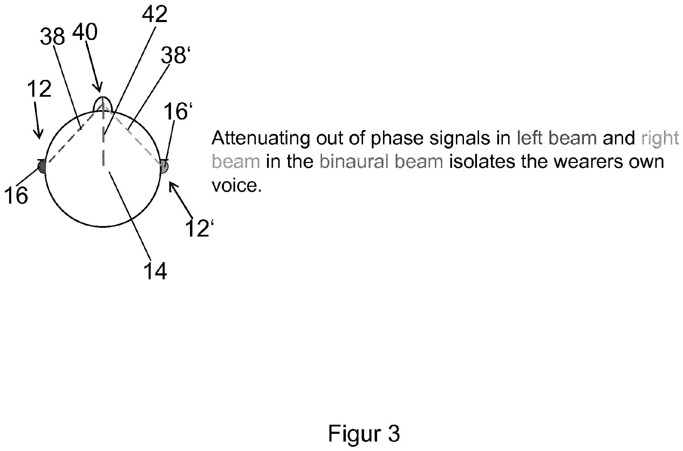

Figure 3 illustrates binaural beam forming according to an embodiment of the disclosure. The microphone 16 at the left ear 12 and the microphone 16' at the right ear 12' represent directional microphone systems, e.g., with front-front or front-rear configuration as shown in fig. 2. Each of the microphones 16 and 16' generates a directional beam 38 and 38', respectively, which points to the mouth 40 of the user 14.

Using binaural link 36, 36' the microphone signals from the left and right directional microphone systems, i.e., left directional beam 38 and right directional beam 38' can be transmitted between the two hearing devices 10 and 10' (c.f. fig. 1). The directional beams 38 and 38' are then used in signal processing units 22, 22' to construct a binaural beam 40 pointing directly forward from the mouth 40 by attenuating the out of phase parts in the two directional beams 38, 38'. Only signals that originate from the junction of the three beams pass through the combined binaural and monaural directional microphone system located at the mouth 40. Therefore, signals derived using the combined binaural and monaural directional microphone system are likely to comprise user's own voice. The signal processing units 22, 22' can isolate the user's own voice by using the combined binaural and monaural directional microphone system, i.e., performing process b).

The signal processing units 22, 22' can extract acoustical parameters from the microphone signals 30, 32, 34, 30', 32', 34' that allow to decide on whether the sound signals originate from a position close to the microphones 16, 18, 20, 16', 18', 20' or far from the microphones 16, 18, 20, 16', 18', 20'. The signal processing units 22, 22' can use the information whether the sound signals originate from a position close to the microphones 16, 18, 20, 16', 18', 20' or far from the microphones 16, 18, 20, 16', 18', 20' to isolate the user's own voice. The information whether the sound signals originate from a position close to the microphones 16, 18, 20, 16', 18', 20' or far from the microphones 16, 18, 20, 16', 18', 20' allows for near field emphasis, i.e., attenuating signals originating from a position far from the microphones 16, 18, 20, 16', 18', 20'. The signal processing units 22, 22' can isolate the user's own voice by using the information regarding the near field emphasis in combination with the combined binaural and monaural directional microphone system, i.e., performing process c). This allows for increasing the reliability of the isolation of the user's own voice.

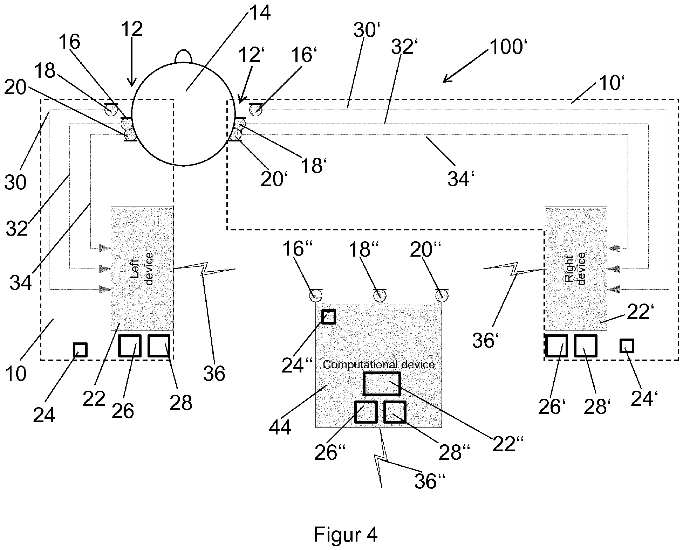

Figure 4 illustrates an embodiment of a hearing system 100' with an auxiliary device 44 and two hearing devices 10, 10' according to an embodiment of the disclosure. The auxiliary device 44 can be arranged at the user, e.g., at the body of the user 14, or can also be external to the user 14. The auxiliary device 44 can for example be a smartphone, tablet PC, hearing device remote control, or the like.

The auxiliary device 44 comprises three microphones 16", 18", 20", a signal processing unit 22", a memory 24", a receiver 26", and a transmitter 28".

The auxiliary device 44 can perform the same functions as each of the hearing devices 10, 10'. The auxiliary device 44, however, comprises a more powerful signal processing unit 22", a larger memory 24" and a larger power source. This allows for reducing the power consumption at the hearing devices 10, 10' if the computations are mainly performed in the signal processing unit 22" of the auxiliary device 44.

The embodiment of the hearing system 100', as shown in figure 4, can have an identical functionality as the embodiment of the hearing system 100 presented in fig. 1 in this case the auxiliary device 44 does not perform any functions. The embodiment of figure 4 can also use the signal processing unit 22" of the auxiliary device 44 in order to perform certain processes in order to isolate the user's own voice.

Therefore, either the microphones 16", 18", 20" of the auxiliary device 44 receive sound signals and generate microphone signals from them or the receiver 26'" of the auxiliary device 44 receives microphone signals 30, 32, 34, 30', 32', 34' via the binaural link 36" from the transmitters 28, 28' of the hearing device 10 and 10'. The microphone signals are then provided to the signal processing unit 22" which performs one or more of the processes a), b), c), and d) in order to isolate the user's own voice.

At least two of the processes in order to isolate the user's own voice are performed by the signal processing units 22, 22', 22" of the hearing system 100'. The outputs of the processes are transmitted between the devices 10, 10' and 44 via the binaural links 36, 36', 36" in order to fuse the results and obtain an improved isolated voice signal. The isolated voice signal is then used by the signal processing units 22, 22', 22" in order to estimate a stress level or cognitive load of the user. Therefore, the signal processing unit estimates the pitch, amplitude and frequency of the user's own voice, i.e., the speech characteristics of the user and analyses them by comparing them to speech characteristics of the user stored in the memory. The stress level or cognitive load can also be estimated from one or more of the following: pitch, frequency, amplitude, speaking rate, variability of pitch, variability of frequency, variability of amplitude, and/or variability of speaking rate of the isolated voice of the user. Furthermore, the speech characteristics of the isolated voice are stored in the memory in order for subsequent use, e.g., for comparing them to isolated voice signals in the future. This allows the hearing system 100' to learn the user's speech characteristics over time.

The signal processing units 22, 22', 22" then adjust the settings of the devices 10, 10' and 44 in dependence of the estimated stress level or cognitive load, e.g., decreasing or increasing overall sound level, activating or deactivating a device, adjusting parameters or the like.

The auxiliary device 44 can also comprise a user interface (not shown) for controlling the hearing system 100' (not shown). The user interface can for example be used to input the current mood of the user 14 estimated by user 14 in order to improve the learning process of the speech characteristics of the user 14 by the hearing system 100'.

The auxiliary device 44 can also comprise an algorithm for own-voice detection. When a hearing-aid wearer's own-voice is detected, their speech can either be analyzed directly in the hearing aid, or transferred to remote analysis in the auxiliary device 44 with automatic own-voice processing. The own-voice processing should allow for transferring of the results to the person's general practitioner or family member in order to be continuously monitoring the cognitive health of the wearer.

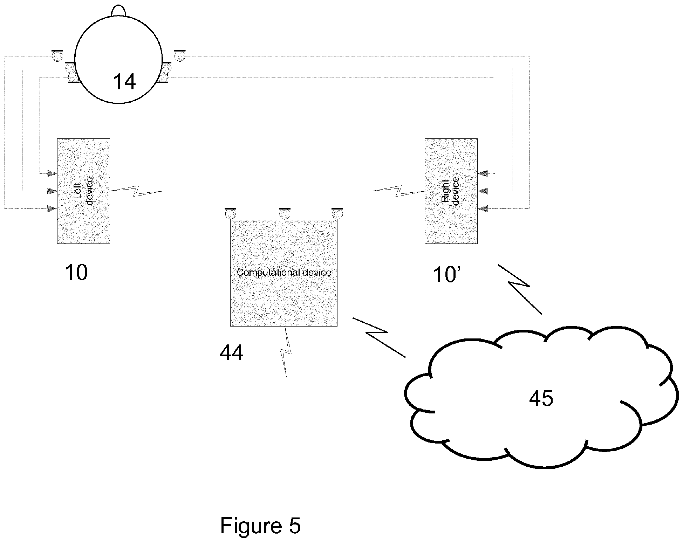

Figure 5 illustrates an embodiment of a hearing system 100' with an auxiliary device 44 and two hearing devices 10, 10' according to the disclosure. The embodiment of figure 5 comprises an auditory input of the hearing aid, which contains the wearers own voice, which is detected and either processed within the hearing aid or not before being wirelessly transferred to a remote system comprising the auxiliary device and/or a cloud system 45. The remote system can perform voice analysis and is accessible to the user 14, medical personal or family members.

A person undergoing a personal development process, where one type of behaviour is encouraged and another type of behaviour is discouraged, may not always be conscious about when (and if) he or she is doing things 'right' and 'wrong', respectively. In such cases, feedback (from others, if they are present and aware of the situation) will typically facilitate the development process. 'Right' and 'wrong' behaviour may in some cases be reflected in the person's speech. Thus, use of certain specific words and phrases may be indicative of the wanted behaviour, while use of other words and phrases may be indicative of the unwanted behaviour. Accordingly, applying a continuous speech recognition algorithm (speech-to-text transformation) to the speech spoken by the person would allow automatically generated feedback to be given to the person when certain words were uttered during daily life. This could be combined with a long-term analysis, where the user would be able to follow the trend in his or her own development. This method could, for example, be relevant for adults participating in a 'lifestyle coaching' process, but it could also be relevant for hearing-impaired children as an assistive tool used during their language development.

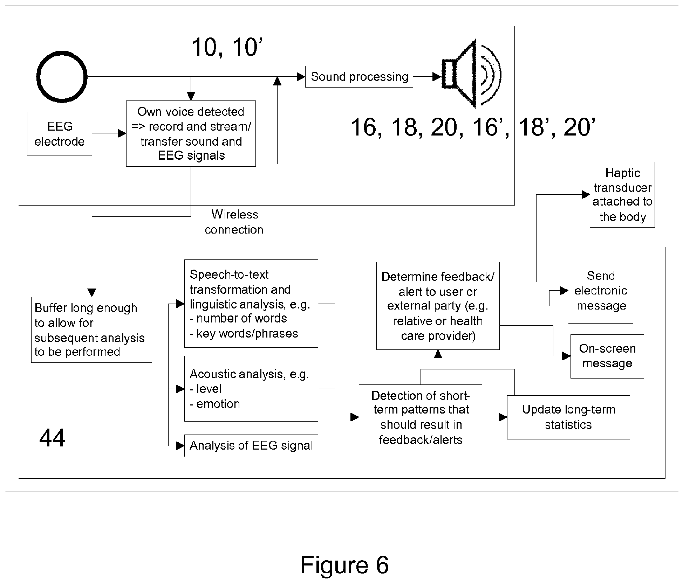

Figure 6 illustrates a hearing device 10, 10' of a hearing system 100' according to an embodiment of the disclosure. The hearing device includes a microphone, an EEG electrode and an own-voice detector (using the microphone signal, the EEG signal, or both as input) and means to transfer the signals to the external device.

Whenever the voice of the user is detected, the sound signal is recorded by the hearing-device microphone and transferred, together with the simultaneously recorded EEG signal, to an auxiliary device 44 (e.g. a smartphone) or a cloud system 45, where further processing is performed.

The processing involves speech recognition (used to perform a signal-to-text transformation/transcription) followed by a linguistic (semantic) analysis (e.g., detection of certain key words, sentences or phrases indicating a positive or negative mood, but various other analyses could be imagined), an acoustic analysis of the speech signal (e.g., determination of sound pressure levels and spectra, and assessment of emotional characteristics), an analysis of the EEG signal (extracting relevant measures), and administration of relevant short- and long-term statistics on the outcomes from the various analyses.

When certain criteria are met, e.g. a consistent change in daily speech/EEG statistics over a period of time, which indicates a mental-health disorder, or the detection of certain words or phrases, indicating a certain wanted or unwanted behaviour, a certain action is taken. In case of a mental health disorder, the action could, for example, be to warn the user via the auxiliary device 44 and/or to send a notice to the general practitioner or a relative. In case of behaviour changes, the action could be to provide the user with immediate auditory, visual or haptic feedback (via the hearing device, the auxiliary device, or a possible third device (a wristband or the like)).

The suggested solution is based on use of a number of already known methods and technologies, for example, the methods to perform own voice detection, speech recognition and the various types of speech analysis.

The concept of the invention is shown in the block diagram in figure 6. The figure aims to illustrate the basic functionality rather than to show the actual implementation (which may be quite different). The optimal way to implement the functionality needs further consideration.

In the example, the hearing device includes an own-voice detection algorithm, which could be based on either the audio signal, the EEG signal (see e.g. patent application EP 13172066.6), or the two signals in combination. When own voice is detected, the speech signal (own voice) as well as the EEG signal are recorded and transferred to an external device (e.g., wirelessly to a smartphone).

The speech signal will be subjected to a speech recognition system, basically enabling a speech-to-text transcription to be made. The text will then be analysed linguistically/semantically to extract relevant outcome measures, e.g.:
- total number of words spoken (counted during a day)
- number of positively loaded words/phrases spoken and number of negatively loaded words/phrases spoken, and the ratio between these numbers
- various other relevant linguistic characteristics

The speech signal will also be subjected to an acoustic analysis, providing data on, e.g.:
- total duration of own speech (during a day)
- distribution of own-voice sound pressure levels
- detection of speech emotions and their distribution

The EEG signal will be analysed to extract levels and distributions of relevant descriptor variables.

When the invention is used as a mental-health monitoring device (cf. problem 1), the system should, on a daily basis, generate data points for all outcome measures indicated above, enabling monitoring the trend over a long period of time and allowing automatic detection of trends away from the 'normal' range. The use of the system should include initial assessment of the 'normal' range, which is expected to be quite individual.

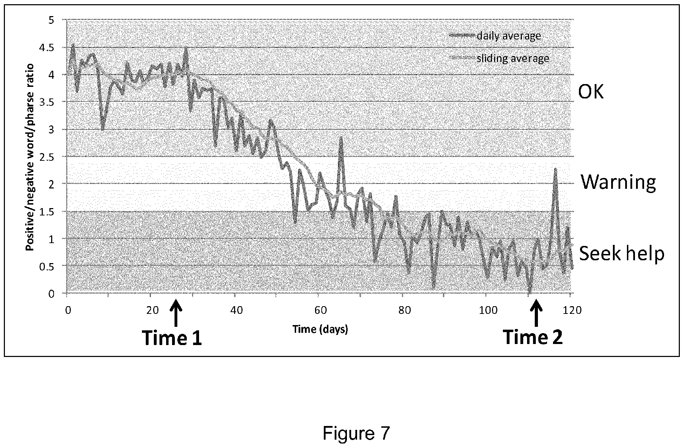

Figure 7 illustrates an example according to an embodiment of the disclosure. The user uses a lot of positively loaded words during a day at Time 1, where he is in his 'normal' condition. At Time 2, his speech is dominated by negatively loaded words, due to the development of a depressive disorder. This is reflected in a (fabricated) outcome measure indicating the ratio between positive and negative words, as shown in figure 7 where Time 1 and 2 are indicated. This figure shows the declining trend (within the expected (and natural) day-to-day variation) when the monitoring is performed over several days.

All outcome measures, based on speech as well as EEG signals, should be combined to determine the appropriate action, which for example could be 'no action' (or positive feedback to the user), a warning to the user, or a direct advice to seek help (possibly in combination with an alert sent automatically to the GP, another health care provider, and/or a relative).

If the invention is used to monitor own behaviour, an instantaneous feedback may be given to the user in certain specific situations (e.g. when certain words/phrases are uttered), It could be either auditory feedback via the hearing device, written feedback via the external device, or haptic feedback via the external device or a possible third device (see figure 6). Another option may be to allow the user to follow the (daily and long-term) stats, e.g. via use of an app, to see the development in personal behaviour. The same feedback options could be relevant if the invention is used to assist in language development in children.

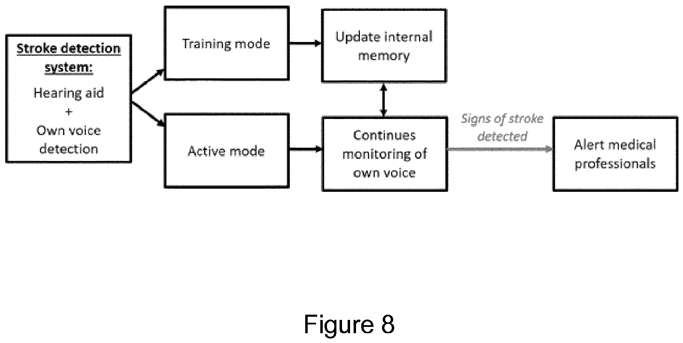

Figure 8 illustrates an example according to an embodiment of the disclosure, as a stroke detection system. The system consists of a hearing device with own-voice detection software. The system has a training mode and an active mode. During the training mode, the wearer is prompted to repeat certain sentences in order to train/update the internal memory with features of natural voice/speech/language. Within the active mode, the natural voice/speech/language is continuously monitored and compared to the internal memory in order to identify any warning signs of a stroke.

This example focuses on the voice/speech/language difficulty that occurs as early warning of a stroke. Natural voice/speech/language processing can already identify health conditions such as dementia, as described above and Parkinson's disease. The stroke detection system is composed of four components: a hearing device, a natural voice/speech/language processing and monitoring system, an embedded test system for baseline and emergency purposes, and an alert, feedback, and advice system when a stroke is detected. The system has the advantage of being body-worn and of being capable of comparing voice/speech/language between baseline (no stroke) and emergency (stroke).
The system is composed of two mandatory components (1-2) and two optional components (3-4; shown in figure 8):
1. **Hearing device**, e.g. a hearing aid, cochlear implant, or bone-anchored hearing system, 'hearable' / personal sound amplifying product intended for people without hearing loss, or any other device that include a microphone, a receiver, and some signal processing, which uses the algorithm for own voice detection implemented in the hearing device. A signal is transferred to the processing and monitoring system, when the algorithm detects the own voice of the user wearing the hearing device.
2. **Natural voice/speech/language processing and monitoring system**: Natural voice, speech, and language is continuously processed and monitored. The monitoring process comprises characteristics of
   a. vocal fold vibration (regularity, modulation depth, and others)
   b. speech production (speech rate, regularity, vowel formants, spectra of s-sounds, and others)
   c. language (correct replication of words, semantic and grammatical analysis, and others)
   Deviations trigger either the component 3 or 4 or an alert to the user.
3. **Embedded test system for baseline and emergency purposes**: The test system asks the user to repeat standardized sentences. When the user first receives the system, he/she repeats the sentences requested by the test system: the test system stores these baseline measures. Baseline measures are triggered once in a while to estimate 'natural variation'. When the natural voice/speech/language processing and monitoring system identifies abnormal voice/speech/language, the embedded test system asks the user to repeat the standardized sentences and compares the emergency sentences to the baseline sentences. (Users at high stroke risk can trigger this action at given intervals. This would also allow detection in situations where the user is not speaking, e.g. when home alone.) This individual matching of baseline and emergency test results gives better accuracy (sensitivity and specificity) in identifying early warning signs of stroke. If the baseline and emergency sentences are too different, 4 is triggered or an alert is given to the user.
4. **Alert, feedback, and advice system**: An alert is sent to the emergency services (e.g. 112 in Europe or 911 in North America), to the closest nurse ward for hospitalized patients, or to a family member. The alert can include a text message, alerting the recipient that the user is suffering a stroke without relying on the user having to give verbal commands whilst suffering the stroke. Alert settings are selected by the user. When the alert is sent, the device provides feedback (e.g. "help is on its way") and advice to its user and carriers.

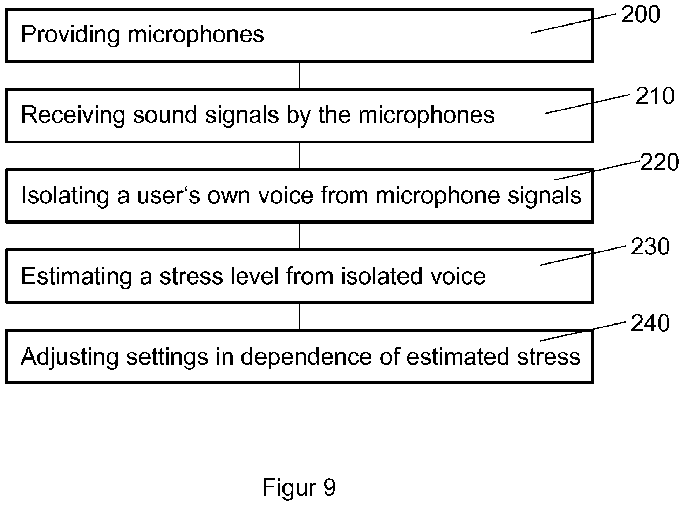

Figure 9 illustrates an embodiment of the signal processing method according to an embodiment of the disclosure. The embodiment of the signal processing method comprises the following steps:

| | |
|---|---|
| 200 | providing at least two microphones at one ear of a user or at least one microphone at each ear of the user, |
| 210 | receiving sound signals by the microphones; |
| 220 | isolating a user's own voice from the microphone signals by way of a combination of at least two of the following processes: |
| | a) applying own voice detectors to the microphone signals, |
| | b) using monaural and binaural directional microphone systems, |
| | c) using monaural and binaural directional microphone systems with near field emphasis, and |
| | d) applying speaker identification models to the microphone signals; |
| 230 | estimating a stress level or cognitive load from the isolated voice and |
| 240 | adjusting settings of a device in dependence of the estimated stress level or cognitive load. |

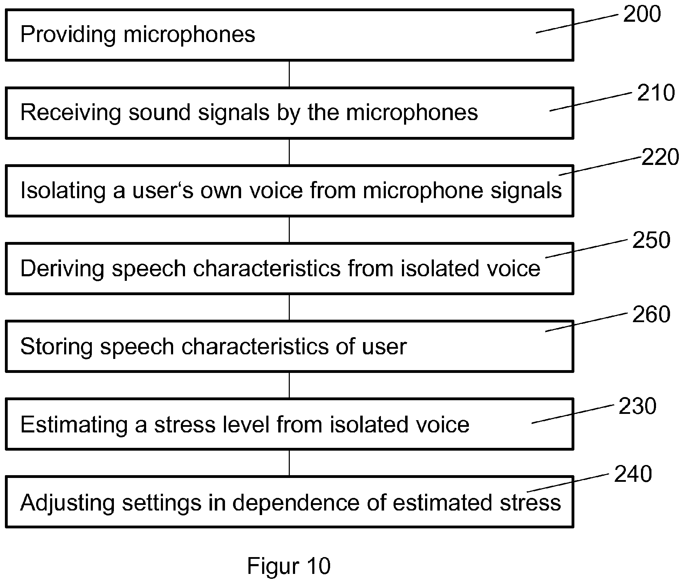

Figure 10 illustrates an embodiment of the signal processing method according to an embodiment of the disclosure. The embodiment of the signal processing method comprises the following steps:

| | |
|---|---|
| 200 | providing at least two microphones at one ear of a user or at least one microphone at each ear of the user, |
| 210 | receiving sound signals by the microphones; |
| 220 | isolating a user's own voice from the microphone signals by way of a combination of at least two of the following processes: |
| | a) applying own voice detectors to the microphone signals, |
| | b) using monaural and binaural directional microphone systems, |
| | c) using monaural and binaural directional microphone systems with near field emphasis, and |
| | d) applying speaker identification models to the microphone signals; |
| 250 | deriving speech characteristics of the user from the isolated voice; |
| 260 | storing the speech characteristics of the user for subsequent use; |
| 230 | estimating a stress level or cognitive load from the isolated voice and |
| 240 | adjusting settings of a device in dependence of the estimated stress level or cognitive load. |

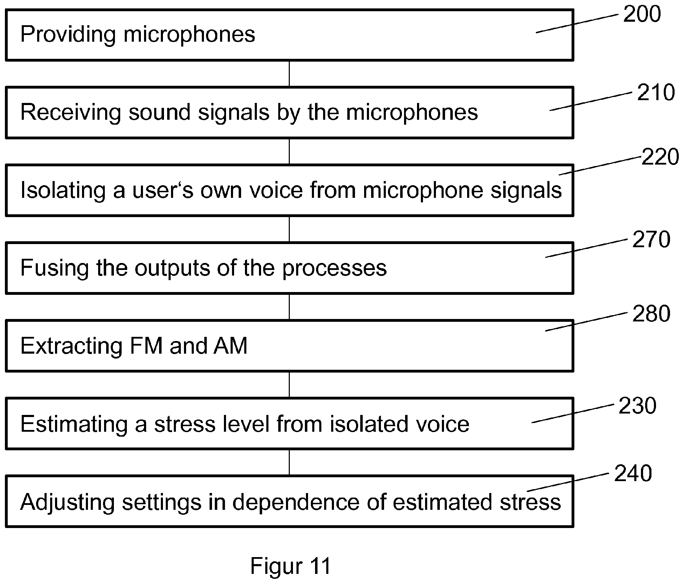

Figure 11 illustrates an embodiment of the signal processing method according to an embodiment of the disclosure. The third embodiment of the signal processing method comprises the following steps:

| | |
|---|---|
| 200 | providing at least two microphones at one ear of a user or at least one microphone at each ear of the user, |
| 210 | receiving sound signals by the microphones; |
| 220 | isolating a user's own voice from the microphone signals by way of a combination of at least two of the following processes: |
| | a) applying own voice detectors to the microphone signals, |
| | b) using monaural and binaural directional microphone systems, |
| | c) using monaural and binaural directional microphone systems with near field emphasis, and |
| | d) applying speaker identification models to the microphone signals; |
| 270 | fusing the outputs of the processes; |
| 280 | extracting frequency modulations and amplitude modulations; |
| 230 | estimating a stress level or cognitive load from the isolated voice by estimating the stress level or cognitive load from the extracted frequency modulations and amplitude modulations and |
| 240 | adjusting settings of a device in dependence of the estimated stress level or cognitive load. |

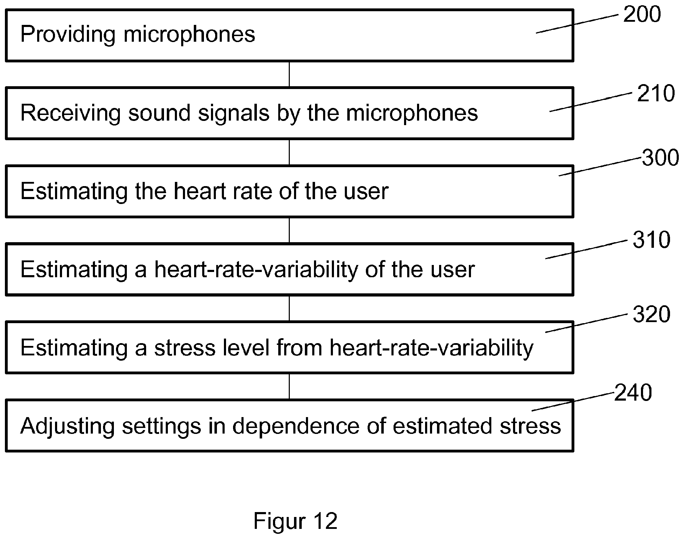

Figure 12 illustrates an embodiment of the signal processing method according to the disclosure. The fourth embodiment of the signal processing method comprises the following steps:

| | |
|---|---|
| 200 | providing at least two microphones at one ear of a user or at least one microphone at each ear of the user, |
| 210 | receiving sound signals by the microphones; |
| 300 | estimating the heart rate of the user based on the microphone signals; |
| 310 | estimating a heart-rate-variability of the user from the heart rate of the user; |
| 320 | estimating a stress level or cognitive load from based on the heart-rate-variability of the user and |
| 240 | adjusting settings of a device in dependence of the estimated stress level or cognitive load. |

The signal processing method monitors the heart rate of the user continuously by receiving sound signals by the microphones. The microphones provided for the fourth embodiment of the signal processing method are preferably arranged between the tympanic membrane and the output transducer of the hearing device. This allows for a better performance as the heart beat can be directly received inside of the ear canal and therefore the heart rate is known to be the user's heart rate, rendering isolation of the user's own voice or signals from the user unnecessary.

The steps of the above mentioned embodiments of the signal processing method can also be separate functions independently performed by separate signal processing units 22, 22', 22" of the hearing systems 100, 100'. The isolation of the user's own voice and the estimation of the stress level or cognitive load can be performed by different signal processing units 22, 22', 22" on different devices 10, 10', 44 in order to improve the performance of the signal processing.

In an aspect, the functions may be stored on or encoded as one or more instructions or code on a tangible computer-readable medium. The computer readable medium includes computer storage media adapted to store a computer program comprising program codes, which when run on a processing system causes the data processing system to perform at least some (such as a majority or all) of the steps of the method described above and in the claims.

By way of example, and not limitation, such computer-readable media can comprise RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Disk and disc, as used herein, includes compact disc (CD), laser disc, optical disc, digital versatile disc (DVD), floppy disk and Blu-ray disc where disks usually reproduce data magnetically, while discs reproduce data optically with lasers. Combinations of the above should also be included within the scope of computer-readable media. In addition to being stored on a tangible medium, the computer program can also be transmitted via a transmission medium such as a wired or wireless link or a network, e.g. the Internet, and loaded into a data processing system for being executed at a location different from that of the tangible medium.

In an aspect, a data processing system comprising a processor adapted to execute the computer program for causing the processor to perform at least some (such as a majority or all) of the steps of the method described above and in the claims.

The steps of the embodiments of the signal processing methods can be implemented in software. The steps of the embodiments of the signal processing methods can be performed on the signal processing units 22, 22', 22" or any other data processing system and the estimated stress levels or cognitive loads can then be transmitted to the devices 10, 10' and 44 in order to allow the signal processing units 22, 22', 22" to adjust the settings of the devices 10, 10' and 44 in dependence of the estimated stress level or cognitive load.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more.

Accordingly, the scope should be judged in terms of the claims that follow.

### REFERENCE SIGNS

- 10, 10': hearing device
- 12, 12': ear
- 14: user
- 16, 16', 16": front microphone
- 18, 18', 18": intermediate microphone
- 20, 20', 20": rear microphone
- 22, 22', 22": signal processing unit
- 24, 24', 24": memory
- 26, 26', 26": receiver
- 28, 28', 28": transmitter
- 30, 30': front microphone signal
- 32, 32': intermediate microphone signal
- 34, 34': rear microphone signal
- 36, 36', 36": binaural link
- 38, 38': directional beam
- 40: mouth of the user
- 42: binaural beam
- 44: auxiliary device
- 45: cloud system
- 100: hearing system

## Claims

1. A signal processing method, comprising:
providing at least two microphones at one ear of a user or at least one microphone at each ear of the user,
receiving sound signals by the microphones;
isolating a user's own voice from the microphone signals by way of a combination of at least two of the following processes:
a) applying own voice detectors to the microphone signals,
b) using monaural and binaural directional microphone systems,
c) using monaural and binaural directional microphone systems with near field emphasis, and
d) applying speaker identification models to the microphone signals;
estimating a stress level or cognitive load from the isolated voice and
adjusting settings of a device in dependence of the estimated stress level or cognitive load.

2. The signal processing method according to claim 1, wherein
the process of applying own voice detectors to the microphone signals comprises one or both of the following processes:
A) determining from the microphone signals the characteristics resulting from the microphones being in the acoustical near-field of the user's mouth and in the far-field of other sources of sound, and
assessing based on these characteristics whether the sound signals originate from the user's own voice or originate from another source,
B) determining from the microphone signals the characteristics resulting from the user's mouth being placed symmetrically with respect to the microphones arranged at the user's head, and
assessing based on these characteristics whether the sound signals originate from the user's own voice or originate from another source
and wherein the process of applying own voice detectors to the microphone signals further comprises
providing a binary flag that represents whether the user's own voice is detected in the microphone signals or a probability value that represents the probability of the user's own voice being present in the microphone signals.

3. The signal processing method according to claim 1 or 2, wherein
the process of using monaural and binaural directional microphone systems comprises
attenuating sound signals that do not have a time delay between the at least two microphones at one ear of the user that matches the time delay from the user's mouth direction, whereby a directional beam is generated.

4. The signal processing method according to claim 3, wherein
the process of using monaural and binaural directional microphone systems further comprises
attenuating out of phase signals in two directional beams, each of which is generated from attenuating the sound signals at one ear of the user, in a binaural beam, such that the binaural beam as a result points directly forward from a mouth position of the user.

5. The signal processing method according to claims 3 or 4, wherein
the process of using monaural and binaural directional microphone systems with near field emphasis comprises
extracting acoustical parameters from the microphone signals that allow to decide on whether the sound signals originate from a position close to the microphones or far from the microphones, and
using the information whether the sound signals originate from a position close to the microphones or far from the microphones to isolate the user's own voice.

6. The signal processing method according to at least one of the claims 1 to 5, wherein
the process of applying speaker identification models to the microphone signals comprises
providing an output for each time-frequency bin of the microphone signals and, selecting only the time-frequency bins that resemble speech characteristics of the user for further processing of the microphone signals.

7. The signal processing method according to claim 6, wherein
the signal processing method comprises
deriving speech characteristics of the user from the isolated voice and storing the speech characteristics of the user for subsequent use.

8. The signal processing method according to at least one of the claims 1 to 7, wherein
a combination of at least two of the processes performed on the microphone signals is done by
fusing the outputs of the processes, and
extracting frequency modulations and amplitude modulations, and estimating of the stress level or cognitive load from the isolated voice is done by
estimating the stress level or cognitive load from the extracted frequency modulations and amplitude modulations.

9. The signal processing method according to at least one of the claims 1 to 8, wherein
the signal processing method comprises
providing at least a microphone at a position between a loudspeaker and tympanic membrane of the user, and wherein
the sound signals received by the at least one microphone at a position between loudspeaker and tympanic membrane of the user are used for isolating a user's own voice.

10. The signal processing method according to at least one of the claims 1 to 9, wherein
the signal processing method comprises
monitoring a heart rate of the user comprising
receiving sound signals by the microphones, wherein
the microphones used to estimate the heart rate of the user are arranged at a position between a loudspeaker and tympanic membrane of the user, and estimating the heart rate of the user based on the microphone signals,
estimating a heart-rate-variability of the user from the heart rate of the user, and estimating the stress level or cognitive load based on the heart-rate-variability of the user.

11. A hearing device comprising:
at least two microphones adapted to be arranged at one ear of a user or at least one microphone adapted to be arranged at each ear of the user for receiving sound signals, and
a signal processing unit adapted to isolate the user's own voice from the microphone signals by way of a combination of at least two of the following processes:
a) applying own voice detectors to the microphone signals,
b) using monaural and binaural directional microphone systems,
c) using monaural and binaural directional microphone systems with near field emphasis, and
d) applying speaker identification models to the microphone signals,
further adapted to estimate a stress level or cognitive load from the isolated voice and furthermore adapted to adjust settings of the hearing device in dependence of the estimated stress level or cognitive load.

12. The hearing device according to claim 11, wherein
the signal processing unit is adapted to apply own voice detectors to the microphone signals by performing one or both of the following processes:
A) determining from the microphone signals the characteristics resulting from the microphones being in the acoustical near-field of the user's mouth and in the far-field of other sources of sound, and
assessing based on these characteristics whether the sound signals originate from the user's own voice or originate from another source,
B) determining from the microphone signals the characteristics resulting from the user's mouth being placed symmetrically with respect to the microphones arranged at the user's head, and
assessing based on these characteristics whether the sound signals originate from the user's own voice or originate from another source,
and wherein the signal processing unit is adapted to provide a binary flag that represents whether the user's own voice is detected in the microphone signals or a probability value that represents the probability of the user's own voice being present in the microphone signals.

13. The hearing device according to claim 10 or 11, comprising a memory adapted for storing a user's speech characteristics resembling a selection of certain time-frequency bins characteristic for the user's speech and storing a set of initial speech characteristics.

14. The hearing device according to at least one of the claims 10 to 13, comprising
a transmitter for transmitting signals to another hearing device, an auxiliary device or a cloud system and
a receiver for receiving signals from another hearing device, an auxiliary device or a cloud system.

15. A hearing system
comprising
at least one hearing device according to claim 14 and at least one auxiliary device comprising
a transmitter for transmitting signals to the hearing device,
a receiver for receiving signals from the hearing device, and
a signal processing unit for processing signals received from the hearing device
or at least two hearing devices according to claim 14 and
adapted to perform the signal processing method according to at least one of the claims 1 to 10.
